# FASCICULE DE BREVET EUROPEEN

(11) **EP 3 700 409 B1**
(45) Date de publication et mention de la délivrance du brevet: **01.12.2021**
(21) Numéro de dépôt: 18796729.4
(22) Date de dépôt: 15.10.2018
(51) Int. Cl.: A61B 5/00, G01N 21/64, A61B 90/00, A61B 5/026, A61B 5/0275

(54) **PROCÉDÉ ET DISPOSITIF DE SUIVI DE LA FLUORESCENCE ÉMISE À LA SURFACE D'UN TISSU BIOLOGIQUE**
VERFAHREN UND VORRICHTUNG ZUR ÜBERWACHUNG DER AN DER OBERFLÄCHE VON BIOLOGISCHEM GEWEBE EMITTIERTEN FLUORESZENZ
METHOD AND DEVICE FOR MONITORING THE FLUORESCENCE EMITTED AT THE SURFACE OF A BIOLOGICAL TISSUE

(30) Priorité: 26.10.2017 FR 1760109
(43) Date de publication de la demande: 02.09.2020
(73) Titulaire: Fluoptics, 38040 Grenoble (FR)
(72) Inventeur: DAURES, Anthony, 92110 Clichy (FR); RIZO, Philippe, 38700 La Tronche (FR)
(74) Mandataire: INNOV-GROUP
(86) Numéro de dépôt international: PCT/FR2018/052553
(87) Numéro de publication internationale: WO 2019/081833

(56) Documents cités:
- WO-A1-2008/081659
- WO-A2-2009/127972
- US-A1- 2010 305 454
- US-A1- 2017 084 012

## Description

L'invention concerne le domaine de l'imagerie médicale. Plus particulièrement, l'invention concerne un procédé et un dispositif de mesure de la fluorescence dans un tissu biologique. L'invention est définie dans les revendications 1 et 15 qui suivent la présente description.

Un dispositif d'imagerie médicale basée sur la fluorescence d'un marqueur est décrit par exemple dans le document FR2989876A2.

L'imagerie de fluorescence permet de donner une information peropératoire quant à la perfusion dans des tissus biologiques, notamment humains. Pour certaines indications médicales, il peut être intéressant de connaitre la vitesse de montée et de descente du signal fluorescent (donc la pente du signal, c'est-à-dire la vitesse d'augmentation et de diminution de l'intensité du signal), ainsi que l'amplitude du signal de fluorescence (qui représente le niveau de perfusion) pour évaluer la perfusion globale du tissu et identifier d'éventuels problèmes veineux ou artériels (chirurgie plastique, plaies et cicatrisations, ...).

En chirurgie reconstructrice, les chirurgiens cherchent aussi à localiser en préopératoire les artères perforantes d'une zone de tissu à prélever (lambeau). En dynamique de fluorescence, ces artères perforantes se caractérisent par :
- un signal de fluorescence qui augmente en premier (la fluorescence apparait en premier au niveau de ces artères avant de diffuser dans leur voisinage), et
- un signal de fluorescence qui augmente rapidement dans les zones où se situent ces artères perforantes.

Il peut donc être utile de faire ressortir ces informations pour repérer, par exemple, les artères perforantes dans un lambeau de l'artère perforante épigastrique inférieure profonde (soit « DIEP flap » ou « Deep Inferior Epigastric Perforator flap » selon l'expression anglo-saxonne).

Aussi, a-t-il été proposé à cette fin un procédé tel que celui décrit dans la publication de brevet WO2009/127972A2. Cependant, la mise en œuvre de ce procédé nécessite notamment de
- choisir la gamme de temps pendant laquelle un algorithme est appliqué (temps prédéterminé à l'avance),
- choisir quel algorithme utiliser (intégrale dans le temps ou étude des dérivés, par exemple),
- choisir une zone de référence afin que les paramètres extraits des images soient normalisés pour le tissu étudié.

D'une part, cette approche est assez contraignante pour les praticiens et, d'autre part, la qualité et la fiabilité de l'interprétation des résultats sont très dépendantes des choix du praticien et peuvent fortement varier d'un patient, d'une étude et/ou d'un praticien à l'autre.

Plus particulièrement, dans l'approche décrite dans la demande de brevet WO2009/127972A2, la détection des vaisseaux perforants est effectuée en calculant sur une durée prédéterminée, pour chaque pixel, une intensité de fluorescence intégrée dans le temps ou une vitesse d'augmentation de l'intensité de fluorescence.

Cette approche utilisant une durée prédéterminée est très restrictive en ce sens qu'il y a une très forte variabilité entre les patients. Les temps d'évolution de la perfusion peuvent être significativement différents d'un patient à un autre sans pour autant que cela soit pathologique ou représentatif d'un problème.

Un but de l'invention est de permettre au praticien de suivre la diffusion d'un marqueur tout en s'affranchissant le plus possible du facteur temps.

Ce but est au moins en partie atteint avec un procédé de suivi, dans le temps, de la diffusion d'un marqueur fluorescent au sein d'un tissu biologique, dans lequel la diffusion du marqueur est avant tout caractérisée par une mesure de la surface au niveau de laquelle est apparue la fluorescence. Cette surface est éventuellement comparée à une surface correspondant à une zone d'intérêt du tissu biologique plus grande et comprenant la surface au niveau de laquelle est apparue la fluorescence. Eventuellement, le signal de fluorescence mesuré est comparé sur une zone observée où il est déjà apparu et une zone qui sera perfusée plus tardivement.

Ainsi, une information sur l'état de la diffusion du marqueur peut être fournie lorsqu'est constatée une évolution significative de la proportion de la surface du tissu biologique sur laquelle le signal représentatif de la fluorescence a atteint un certain niveau.

Plus particulièrement ce procédé comprend au moins une acquisition d'images de fluorescence, à l'aide d'une caméra, chaque acquisition d'images de fluorescence comprenant la capture et la mise en mémoire d'au moins une image de fluorescence sur une zone de tissu biologique et chaque image de fluorescence correspondant à un ensemble de pixels, une valeur relative d'un signal représentatif de l'intensité de l'émission de fluorescence en un point de la zone du tissu biologique étant associée à au moins certains pixels, donc éventuellement à chaque pixel de l'image de fluorescence.

Ce procédé comprend en outre, au cours d'une même perfusion du marqueur fluorescent, une étape consistant à déterminer automatiquement, après comparaison de la surface de diffusion du marqueur biologique mesurée sur au moins deux images de fluorescence, si tout ou partie d'au moins l'une de ces images de fluorescence pour lesquelles la surface de diffusion a été mesurée doit être affichée, ou non.

Ainsi, grâce au procédé selon l'invention, le critère décisif pour fournir et afficher une image de la diffusion est la mesure de la surface, sur cette image ou une zone de cette image, pour laquelle la fluorescence est visible avec une intensité prédéterminée. Un logiciel pour la mise en œuvre du procédé selon l'invention, peut ainsi traiter automatiquement des images de fluorescence en vue de leur affichage, cet affichage étant conditionné par la dimension d'une surface représentant la diffusion dans l'image. Il est alors possible de n'afficher que des images sur lesquelles cette dimension a varié de manière suffisamment significative pour avoir un intérêt pour le praticien. A contrario, dans les procédés de l'art antérieur basés sur des temps d'acquisition, une ou plusieurs images d'une séquence pouvaient être affichées sans qu'une variation significative de la diffusion soit observable ou au contraire avec une trop grande variation entre deux images successives pour donner au praticien une information utile. Le document US2017/0084012 décrit un système d'affichage d'images de fluorescence.

Le procédé selon l'invention comporte en outre au moins l'une des caractéristiques correspondant aux revendications 2 à 14, chacune considérée isolément ou en combinaison d'une ou plusieurs autres.

L'invention concerne également un dispositif selon l'une des revendications 15 et 16.

D'autres caractéristiques et avantages de l'invention apparaîtront à la lecture de la description détaillée qui suit, ainsi que sur les dessins annexés. Sur ces dessins :
- la figure 1 représente schématiquement un mode de réalisation d'un dispositif selon l'invention ;
- la figure 2A représente l'intensité d'un signal de fluorescence, dans deux régions différentes d'un tissu biologique d'intérêt (par exemple un lambeau de l'artère perforante épigastrique inférieure profonde) pour chaque nième image, et la figure 2B représente l'intégrale de cette intensité, en fonction du nombre d'images sommées, ce nombre n'étant pas fixé à l'avance ;
- la figure 3 présente un enchainement d'étapes, selon un exemple de procédé conforme à l'invention, mises en œuvre pour visualiser les zones où se trouvent potentiellement des perforantes ;
- la figure 4 présente une comparaison entre, un exemple de résultat obtenu à l'aide d'un procédé basé sur une séquence temporelle prédéfinie et un exemple de résultat obtenu à l'aide d'un procédé basé sur une analyse des surfaces de diffusion ;
- la figure 5 représente un exemple de mode d'affichage de résultats obtenus à l'aide d'un procédé conforme à l'invention ;
- Les figures 6 et 7 montrent comment sont identifiées des zones correspondant potentiellement à des perforantes.

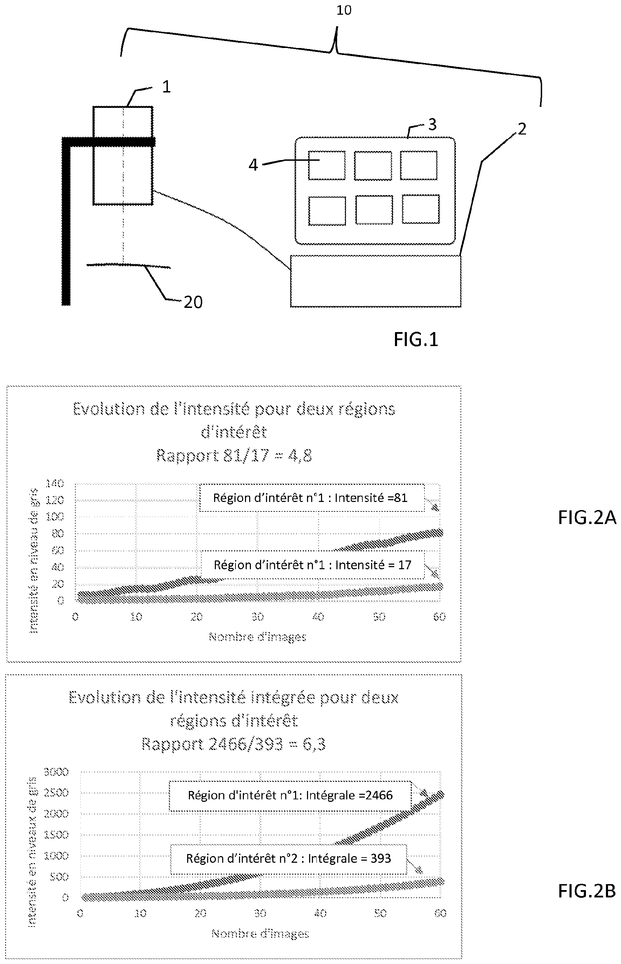

Un exemple de mode de réalisation d'un dispositif 10 de suivi de la fluorescence émise à la surface d'un tissu biologique 20 est représenté sur la figure 1.

Ce dispositif 10 comporte une sonde 1 avec une caméra, dite « de fluorescence » ou « infra-rouge », pour capturer des « images de fluorescence » (dans le proche infra-rouge ou plus généralement dans les longueurs d'ondes détectées par cette caméra « de fluorescence »). Autrement dit, cette caméra est dotée d'au moins un capteur adapté pour capter des images dans le proche infra-rouge ou plus généralement dans les longueurs d'ondes émises par des marqueurs fluorescents. Cette caméra permet donc de réaliser une image de la lumière de fluorescence émise par le fluorophore à la surface de la zone de tissu biologique 20 considérée.

Dans ce document, on nomme « image de fluorescence », une image du signal de fluorescence émis à la surface d'un tissu biologique 20 à observer, capturée à l'aide d'une caméra « de fluorescence » et on nomme « image courante » ou « image de contexte », une image extraite (directement, sans intégration ou sommation avec une ou plusieurs autres images) d'une vidéo réalisée à l'aide d'une caméra de la sonde 1.

Selon une variante, la sonde 1 comporte une première caméra de fluorescence pour capturer des images de fluorescence (par exemple dans le proche infra-rouge) et une deuxième caméra pour capturer des images dans le visible. Dans ce cas, des « images courantes » peuvent être réalisées soit dans la gamme de longueurs d'ondes détectées par la caméra de fluorescence (à l'aide d'une source de lumière dans la gamme de longueurs d'ondes détectées par la caméra de fluorescence, par exemple à l'aide d'une source laser ou de diodes électroluminescentes émettant dans le proche infra-rouge), soit dans le visible.

Le dispositif 10 comporte également un ordinateur 2 pour enregistrer et stocker les images capturées par chaque caméra, ainsi que pour réaliser un traitement des images de fluorescence et éventuellement des images courantes.

Le dispositif 10 comporte aussi des moyens de visualisation et d'affichage 3 (écran) d'images 4 après traitement.

La sonde 1 est par exemple connectée à l'ordinateur 2.

La sonde 1 comprend également une source d'excitation (laser par exemple ou LED) adaptée pour émettre un rayonnement d'excitation d'un marqueur de fluorescence ou fluorophore.

La sonde 1 est avantageusement maintenue, à l'aide d'un bras de support 5, de manière stable et à une distance constante de la scène comprenant la zone de tissu biologique 20 à observer et étudier (il peut toutefois y avoir un léger déplacement de la scène du fait notamment de la respiration du patient, mais dans ce cas un recalage des images les unes par rapport aux autres permet de corriger les éventuels artefacts introduits par ce déplacement).

Un traceur ou marqueur fluorescent, ou encore fluorophore, est injecté en intraveineuse sous forme de bolus (c'est-à-dire de manière rapide et brève). Le signal d'émission du fluorophore est capté par la caméra « de fluorescence » de la sonde 1 et est enregistré, sous forme d'images de fluorescence, au cours du temps en laissant la source d'excitation allumée.

Un logiciel analyse alors en temps réel les images de fluorescence enregistrées afin :
- De déterminer le début de la montée du signal de fluorescence, c'est-à-dire l'instant T₀ à partir duquel le signal de fluorescence commence à augmenter (voir plus loin comment cet instant T₀ est déterminé).
- De procéder à une sommation d'images de fluorescence à partir du temps T₀.
- De normaliser l'image résultant de la sommation par rapport au maximum d'intensité dans cette image,
- De déterminer le pourcentage du nombre de pixels auxquels est associé un signal supérieur ou égal à une seuil prédéterminé,
- D'afficher l'image résultant de la sommation, si le pourcentage du nombre de pixels auxquels est associé un signal supérieur ou égal à un seuil prédéterminé, présente une variation (en augmentation ou en diminution) supérieure ou égale à un seuil prédéfini ou précalculé,
- De coloriser éventuellement l'image normalisée avant de l'afficher (étape précédente).

Un exemple de mise en œuvre du procédé selon l'invention est décrit ci-dessous de manière plus détaillée.

Pour cet exemple, les notations suivantes sont utilisées :
T₀ : instant considéré comme représentatif du début de l'augmentation de la diffusion d'un marqueur dans la zone de tissu biologique observée.
ΔT : intervalle de temps correspondant à une durée d'acquisition.
F : fréquence d'acquisition des images par la caméra de fluorescence.
I_{Ref} : Image de référence, calculée en début d'acquisition, avant T₀ et qui peut être utilisée pour être soustraite à une ou plusieurs images nouvellement acquises, après que celle-ci ait été calculée.
Ii(Y0), avec i un entier supérieur ou égal à 1 : Image obtenue avant T₀ ou à l'instant T₀.
I'(Y0) : Image intégrée obtenue par sommation de plusieurs images Ii(Y0).
I'_{NORM}(Y0) : Image obtenue par sommation de plusieurs images Ii(Y0), normalisée par son propre maximum.
Ii(Y1), avec i un entier supérieur ou égal à 1 : Image acquise après T₀ et sommée à l'image intégrée déjà obtenue, I'(Y0) ou I'(Y1).
I'(Y1) : Image intégrée obtenue par sommation de l'image I'(Y0) avec une ou plusieurs images Ii(Y1).
I'_{NORM}(Y1) : Image I'(Y1) normalisée par son propre maximum et ayant une proportion supérieure ou égale à Y1, du nombre de pixels correspondant à une intensité relative du signal supérieure à une valeur seuil prédéfinie X%.
Y0 : proportion de pixels dans I'_{NORM}(0) correspondant à une intensité relative du signal de fluorescence supérieure à une valeur seuil prédéfinie de X%.
Yj, Yj+1, ... ,Yz : proportions de pixels correspondant à une intensité relative du signal de fluorescence supérieure à une valeur seuil prédéfinie de X%, dans des images intégrées et normalisées I'_{NORM}(Yj), I'_{NORM}(Yj+1),.... ,I'_{NORM}(Yz), successives.

Le praticien injecte un fluorophore (par exemple de l'ICG - acronyme de Indocyanine-Green) sous forme de bolus.

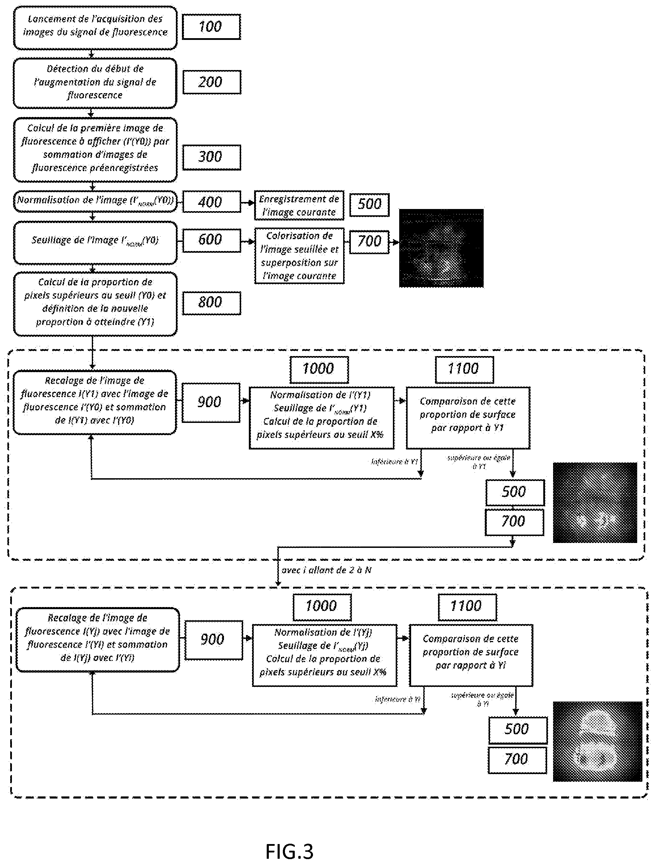

Avant ou au moment de l'injection de fluorophore, un praticien lance une acquisition (Etape 100 de la figure 3). Ainsi, la sonde 1 est allumée et une vidéo est réalisée en continu à l'aide de la caméra de fluorescence. Des images (ne comportant pas nécessairement encore de signal de fluorescence) sont extraites de la vidéo avec une fréquence F. Les données correspondant à ces images captées par la caméra de fluorescence et extraites de la vidéo sont transmises à l'ordinateur 2.

Ces images sont stockées ou enregistrées. Par exemple, elles sont stockées dans une mémoire tampon circulaire, c'est-à-dire une mémoire tampon de taille T fixe. La taille T est choisie pour correspondre à un nombre prédéfini d'images stockées. Par exemple, si les images sont extraites à une fréquence F de vingt images de fluorescence par seconde, on peut choisir la taille T de la mémoire tampon circulaire de manière à ce qu'elle corresponde à 60 images acquises pendant un temps d'intégration correspondant à une plage temporelle de ΔT=3 secondes (T=F^{∗} ΔT =20^{∗}3 = 60 images). Lorsque cette mémoire tampon est pleine, l'image la plus ancienne est supprimée pour pouvoir y ajouter l'image la plus récemment extraite de la vidéo. On a donc une mémoire tampon circulaire (c'est-à-dire de type « premier entré, premier sorti », soit en anglais « first in, first out »).

Au niveau de l'ordinateur 2, un logiciel analyse en temps réel ces données qu'il reçoit de la sonde 1. Plus particulièrement, ce logiciel réalise un premier calcul permettant de détecter l'émergence du signal de fluorescence par rapport au bruit (Etape 200 de la figure 3).

L'émergence du signal de fluorescence est liée au produit fluorescent injecté et correspond au premier point, près de la surface qui est atteint par le marqueur fluorescent en circulation. La durée entre l'injection du bolus de marqueur et l'apparition du premier signal à T₀ est très variable d'un patient à un autre et ne peut pas être prédéterminée. Cette durée peut dépendre du métabolisme du patient sur lequel est faite l'observation. Si le temps T₀ n'est pas obtenu après une durée qui peut être estimée comme pathologique (30 secondes par exemple) le processus est éventuellement arrêté.

Plusieurs méthodes peuvent être envisagées (éventuellement en combinaison d'une ou plusieurs autres méthodes, ou indépendamment les unes des autres), pour détecter le début de la montée du signal de fluorescence, c'est-à-dire l'apparition et le début de l'augmentation du signal de fluorescence.

Selon une méthode, on considère que le signal de fluorescence apparaît lorsqu'on obtient, sur un pixel ou un groupe de pixels d'une image, un signal de fluorescence supérieur ou égal au niveau moyen de l'intensité correspondant à ce pixel ou à ce groupe de pixels (ce niveau moyen étant calculé sur une image de référence) plus, par exemple, trois fois l'écart type de ce niveau. En effet, avec un bruit gaussien, la probabilité pour que le signal moyen d'un pixel sorte de sa valeur moyenne plus trois fois l'écart type est faible.

Cette hypothèse est par exemple considérée vraie pour les premières images de l'acquisition pour lesquelles aucune réelle émission de fluorescence n'est encore présente dans l'image, alors que par ailleurs la scène et les conditions d'illumination restent fixes. Dans ce cas de figure, l'écart type sur la valeur de l'intensité du signal associée à un pixel ou à une moyenne d'un groupe de pixels peut être mesuré en calculant l'écart type de l'intensité du signal associée à ce pixel ou à cette moyenne de pixels obtenue sur quelques premières images au lancement de l'acquisition (par exemple sur 10 images).

Selon une autre méthode, on considère que le signal de fluorescence apparaît lorsqu'on obtient, sur un pixel ou sur un groupe de pixels d'une image, un signal de fluorescence supérieur ou égal à, par exemple, trois fois (ou une autre valeur S multiplicative) la valeur moyenne du signal sur ce pixel ou sur ce groupe de pixels, calculée sur les quelques premières images de l'acquisition, après l'injection de l'ICG (par exemple 3 à 10 images), mais avant qu'un signal de fluorescence significatif ne soit constaté dans l'image. Ainsi, le logiciel analyse les images sur une série de W premières images. Par exemple, le logiciel analyse une série de dix images (W=10). Plus particulièrement, le logiciel calcule une image moyenne I_{Ref} en faisant, pour chaque pixel ou groupe de pixels, une moyenne sur les dix premières images du signal correspondant au pixel ou au groupe de pixels considéré. On appelle M_{init(i,j)} cette valeur moyenne pour un pixel situé aux coordonnées i sur les lignes de l'image et j sur les colonnes de l'image. On appelle Mᵢₙᵢₜ₍ₖ₎ la valeur moyenne pour le k^{ième} groupe de pixels d'une image. Alors que les images continuent d'être transmises à l'ordinateur 2, et enregistrées à la fréquence F dans la mémoire tampon circulaire en ne conservant que les T images les plus récentes, le logiciel compare les intensités M_{(i,j)} par pixel et/ou les intensités M₍ₖ₎ par groupe de pixel de ces images. Le logiciel détecte l'apparition de signal lorsque l'intensité M_{(i,j)} d'un pixel ou M₍ₖ₎ d'un groupe de pixels dans une image excède d'un facteur S fois la valeur M_{init(i,j)} associée aux coordonnées de ce pixel ou respectivement Mᵢₙᵢₜ₍ₖ₎ associée au k^{ième} groupe de pixels dans l'image moyenne précédemment I_{Ref}. Par exemple, S = 3. Alors, lorsque *M*_{(*i,j*)} *> S* × *M*_{*init*(*i,j*)}*,* on considère que le temps T₀ est atteint et que la montée de fluorescence a commencé (Etape 200 de la figure 3).

En outre, comme mentionné ci-dessus, une image de fluorescence I_{Ref} a été calculée, qui servira d'image d'origine, c'est-à-dire d'image de fluorescence de référence, avec d'éventuels résidus d'auto-fluorescence, de fluorescence ou de fond induit par une excitation voulue ou non (éclairage de la salle d'opération par exemple). Alors, cette image moyenne I_{Ref} ainsi calculée sur quelques-unes des premières images successives permet d'obtenir une image de référence I_{Ref} qui est soustraite à chaque nouvelle image de fluorescence Ii(Yj).

Selon encore une autre méthode, on considère que le signal de fluorescence apparaît lorsqu'on obtient un signal supérieur ou égal à une intensité moyenne M_{init(i,j)} ou Mᵢₙᵢₜ₍ₖ₎ (obtenue pour un pixel ou un groupe de pixels), calculée sur quelques premières images de l'acquisition (par exemple 3 à 10 images), à laquelle on ajoute un facteur S'. Par exemple, S'= 10. Alors, lorsque M(i,j) > M_{init(i,j)} + S', on considère que le temps T₀ est atteint et que la montée de fluorescence a commencé.

Selon encore une autre méthode, on considère que le signal de fluorescence apparaît lorsqu'on obtient un signal supérieur ou égal à une intensité moyenne M_{init(i,j} ou Mᵢₙᵢₜ₍ₖ₎ (obtenue pour un pixel ou un groupe de pixels), calculée sur quelques premières images de l'acquisition (par exemple 3 à 10 images), plus une valeur G correspondant à des niveaux de gris (par exemple, G correspond à 15 niveaux de gris).

Quelle que soit la méthode, dès que le début de l'augmentation de la fluorescence est détecté, le signal de fluorescence est suffisant pour qu'un recalage des images les unes par rapport aux autres soit possible. Le logiciel recale alors entre elles les images de fluorescence nouvellement acquises, pour la zone de tissu biologique à observer, par extraction dans la vidéo réalisée en continu. Ce recalage des images de fluorescence entre elles peut se faire à l'aide d'un algorithme standard de recalage d'images. Cependant, on privilégie les algorithmes de recalage à au moins six degrés de liberté (rotations + translations), avec des points singuliers, des flux optiques, etc.

A l'instant T₀, le logiciel somme, de pixel à pixel, la valeur du signal représentatif de l'intensité de l'émission de fluorescence associée à chaque pixel et correspondant à un point de la zone du tissu biologique 20 observée. Cette somme est effectuée par le logiciel sur l'ensemble des images Ii(Y0) stockées (ou préenregistrées), par exemple dans la mémoire tampon circulaire (Etape 300 de la figure 3). Ce calcul revient à faire une intégrale des signaux de fluorescence reçus au niveau de chaque pixel, dans un intervalle de temps ΔT= 3 secondes précédant T₀ dans le cas de notre exemple. Alternativement, la somme est effectuée sur un certain nombre d'images Ii(Y0) stockées (ou préenregistrées), ce nombre étant déterminé, non pas en référence à un intervalle de temps ΔT correspondant à une durée d'acquisition, mais en référence à un pourcentage de pixels dans l'image supérieur ou égal à un seuil prédéfini.

Ce calcul intégratif permet de faire ressortir les pixels associés à une valeur de l'intensité qui a fortement augmenté dans un intervalle de temps. Ceci ressort clairement du diagramme des figures 2A et 2B. Sur la figure 2A sont représentées les intensités pour deux dynamiques de signaux de fluorescence dans deux zones ou régions d'intérêt d'une image de fluorescence : une zone d'intérêt dans laquelle la fluorescence monte plus vite (courbe du dessus) et une zone d'intérêt dans laquelle la fluorescence monte moins vite (courbe du dessous). Sur la figure 2B sont représentées les intensités intégrées, correspondant respectivement aux intensités de la figure 2A. Un calcul intégratif revient à calculer l'intégrale sous chacune des courbes de la figure 2A. Donc, plus la différence entre les deux dynamiques est grande, plus la différence entre les aires sous les courbes correspondantes croit au cours du temps, c'est-à-dire avec le nombre d'images de fluorescence ainsi sommées. Le rapport entre les deux intégrales est en effet plus important que celui entre deux intensités à un temps donné (deux points de même abscisse respectivement sur chacune des courbes). Le calcul intégratif permet donc de mieux faire ressortir les zones dans lesquelles la fluorescence a monté rapidement au cours de l'intervalle de temps sur lequel l'intégration est réalisée. Avec l'exemple représenté sur les figures 2A et 2B, l'intégrale sur les soixante premières images présente un rapport entre les deux courbes de 6,3, tandis que le rapport des intensités correspondantes à la soixantième image est de 4,8.

L'image de fluorescence sommée (intégrée) I'(Y0) est ensuite normalisée (Etape 400 de la figure 3) par son propre maximum dans l'image (on peut d'abord appliquer un filtre médian ou toute autre technique pour supprimer les points chauds avant normalisation) pour obtenir une image 1'_{NORM}(Y0). Autrement dit, pour cette image, l'intensité relative du signal de fluorescence associé à chaque pixel est rapportée par exemple sur une échelle de 0 à 100%, 100% correspondant à l'intensité relative du signal de fluorescence maximale constatée pour cette image. Cette étape est automatique. Il n'est donc plus nécessaire pour le praticien de choisir une zone de référence. On évite ainsi que le praticien ne fasse une erreur liée à son choix.

Selon une autre méthode l'image de fluorescence sommée I'(Y0) est normalisée par une valeur de seuil prédéfinie dépendante du contenu de l'image. Par exemple on pourra normaliser l'image I'(Y0) par un x^{ième} percentile de l'histogramme de l'image I'(Y0). Avec x égal à 95 cela revient à normaliser l'image par la valeur d'intensité d'un pixel tel que 5% des pixels de l'image ont une intensité supérieure à la sienne.

Selon encore une autre méthode, l'image de fluorescence sommée I'(Y0) est normalisée par une valeur de seuil prédéfinie indépendante du contenu de l'image. Par exemple on pourra normaliser I'(Y0) par une valeur de seuil considérée comme témoignant, sur une image de fluorescence, d'un bon niveau de perfusion. Cette valeur est avantageusement définie en fonction de conditions d'utilisation qui peuvent varier. Par exemple, elle peut être réévaluée notamment en fonction de la distance entre la sonde 1 et la surface du tissu biologique observé, de la puissance de la source d'excitation de la fluorescence, de paramètres relatifs à la caméra (temps d'exposition, gain, gamma, etc.), de la dose de marqueur injectée et des conditions d'éclairage extérieur. L'image de fluorescence sommée I'(Y0) peut ainsi être normalisée par cette valeur seuil multipliée par le nombre d'images sommées dans I'(Y0). En prenant en considération tous ces paramètres, on permet à un utilisateur de travailler dans des conditions différentes mais qui ne biaisent nullement le résultat et qui offrent plus de reproductibilité.

Puis cette image de fluorescence normalisée I'_{NORM}(Y0) est seuillée pour ne conserver que les pixels correspondant à une intensité relative du signal de fluorescence supérieure à une valeur seuil prédéfinie X% (Etape 600 de la figure 3). Par exemple X%=50%. Autrement dit, la valeur du seuil X% correspond à une valeur au-delà de laquelle, on considère utile d'exploiter l'information visuellement. X% correspond par exemple à un seuil en deçà duquel l'information sur la fluorescence n'est pas considérée comme significative d'un signal qui augmente précocement et/ou rapidement. La proportion Y0 du nombre de pixels correspondant à une intensité relative du signal supérieure à cette valeur seuil prédéfinie X% est également calculée (Etape 800 de la figure 3). Eventuellement, la proportion suivante à atteindre Y1 est également déterminée à cette étape, par exemple Y1 est égale à deux fois Y0 ou Y1=Y0+5% (alternativement, Y1 est défini avant de lancer l'acquisition).

En choisissant des paramètres Y0,... ,Yj, ... Yz croissants (par exemple 5%, 10%, 20%, 30%, 40%, 50%) on n'affiche une nouvelle image sommée que lorsque l'on observe une augmentation du nombre de pixels excédant X%. Chaque nouvelle image de sommation s'affiche donc lorsque l'on constate une expansion de la surface des pixels excédant X% entre Yj et Yj+1. L'incrément entre Yj et Yj+1 n'est pas nécessairement constant. Par exemple, il peut être avantageux au début de l'acquisition de mieux discriminer le signal correspondant à des vaisseaux perforants, et surtout de ne pas laisser passer une information du fait du choix d'un intervalle de surface trop grand. Alors, par exemple, Y1 peut être égal à Y0 + 5 % puis Y2 égal à Y1 + 7.5%, puis Y3 égal à Y2 + 10 %, puis Y4 égal à Y3 + 10%, puis Y5 égal à Y4 + 10%.

Ainsi, grâce à l'invention on prend automatiquement en considération le niveau de perfusion dans la zone de tissu biologique d'intérêt au temps T₀ ainsi que les différences de vitesse de perfusion notables entre patients. On n'affiche par ailleurs aucune image redondante.

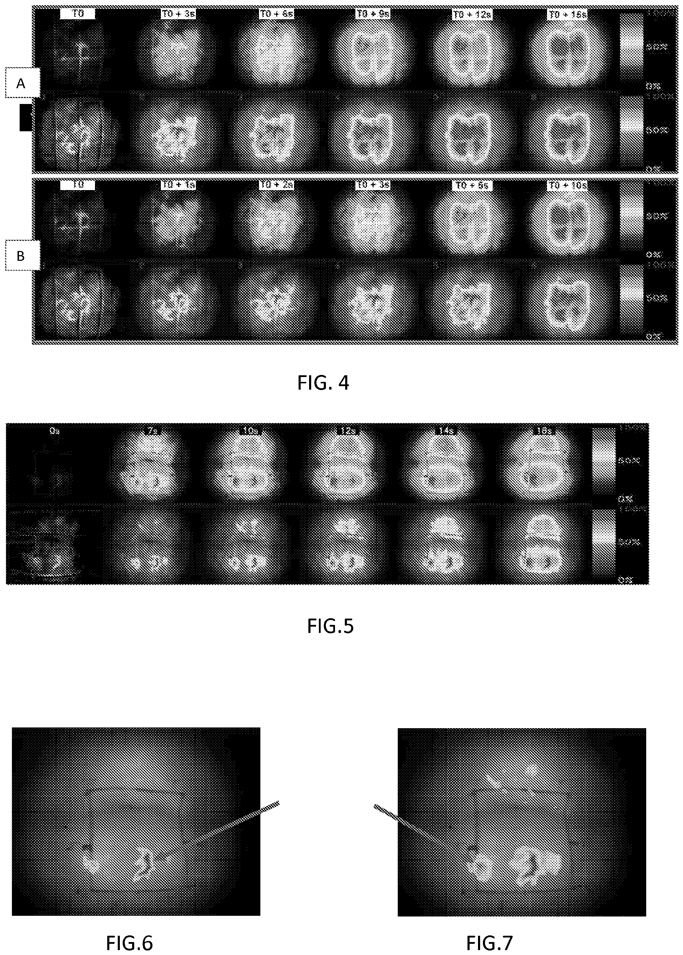

La figure 4 illustre ce principe. Sur le résultat A, où une nouvelle image est affichée avec un intervalle de temps prédéfini, on constate que l'information n'est pas assez précise et discriminée entre T₀ et T₀+6s, période au cours de laquelle le signal a évolué rapidement. Par ailleurs les images affichées après T₀+9s ne sont plus pertinentes et parfois même pratiquement redondantes (comme c'est le cas pour T₀+12s et T₀+15s par exemple).

Par contre, comme le montre le résultat B, en raisonnant sur les surfaces de pixels excédant un seuil d'intensité défini, il est possible de garantir que toute nouvelle image affichée apporte une information supplémentaire par rapport à la, ou aux précédentes. Par ailleurs ce résultat apporte une information plus détaillée et précise entre T₀ et T₀ +5s, permettant de mieux localiser la ou les vaisseaux perforants d'intérêt.

L'image de fluorescence normalisée et seuillée I'_{NORM}(Y0) est aussi colorisée en appliquant une table de conversion des couleurs (Etape 700 de la figure 3). Cette colorisation permet de mettre l'accent sur les fortes valeurs des intégrales de signaux et constitue une aide à la localisation. La table de conversion (« look-up-table » en anglais) peut être d'un type connu ( fausses couleurs, passage en négatif, etc.)

L'image courante correspondant à T₀ (dans le cas où celle-ci est réalisée dans la gamme de longueurs d'ondes détectées par la caméra de fluorescence) est également enregistrée, recalée et archivée (Etape 500 de la figure 3) pour l'afficher en fin d'acquisition, avec les images suivantes qui seront calculées, pour un suivi temporel de l'évolution de la fluorescence, comme ce sera expliqué plus loin. En particulier, l'image de fluorescence normalisée et seuillée I'_{NORM}(0) sera superposée à cette image courante correspondant à T₀.

A partir de ce temps T₀ ainsi défini, le logiciel récupère à un temps T₁ (la durée entre T₀ et T₁ étant déterminé par la fréquence F mentionnée plus haut), une nouvelle image de fluorescence Ii(Y1), la recale par rapport à l'image I(Y0) ou I'(Y0) et la somme directement à l'image I'(Y0) déjà calculée (Etape 900 sur la figure 3), pour obtenir une image sommée I'(Y1). Comme précédemment, l'image I'(Y1) est normalisée pour obtenir une image I'_{NORM}(Y1) et l'image normalisée I'_{NORM}(Y1) est seuillée par rapport à la valeur seuil X%. La proportion P(Y1)% du nombre de pixels correspondant à une intensité relative du signal supérieure à cette valeur seuil prédéfinie X% est également calculée (Etape 1000 de la figure 3) afin de la comparer à la valeur Y1 définie ou précédemment déterminée (Etape 1100 de la figure 3).

Si la proportion P(Y1)% est inférieure à la valeur Y1, l'image suivante Ii+1(Y1) extraite de la vidéo à la fréquence F est recalée et sommée comme I(Y1) pour obtenir une nouvelle image I'(Y1). L'image I'(Y1) résultant de la sommation est normalisée, pour obtenir une nouvelle image I'_{NORM}(Y1) qui est seuillée comme précédemment. La proportion P(Y1)% du nombre de pixels correspondant à une intensité relative du signal supérieure à la valeur seuil prédéfinie X% est calculée et comparée à la valeur Y1 définie ou précédemment déterminée. Si nécessaire, ce processus est répété jusqu'à ce que la proportion P(Y1)% du nombre de pixels correspondant à une intensité relative du signal supérieure à cette valeur seuil prédéfinie X% soit supérieure ou égale à Y1. L'image résultant de ces opérations est donc notée I'_{NORM}(Y1).

Lorsque la proportion P(Y1)% est supérieure ou égale à la valeur Y1, l'image courante est enregistrée et l'image I'_{NORM}(Y1) est colorisée en vue de la superposer à l'image courante, de manière analogue au processus appliqué à l'image I'_{NORM}(Y0).

Le processus est poursuivi de manière semblable afin d'obtenir des images I'_{NORM}(Yj) ; avec j de 1 à Z.

Le processus s'arrête quand on a enregistré Z images I'_{NORM}(Yj) (avec Z un nombre d'images défini comme étant suffisant à l'analyse des vaisseaux perforants, par exemple 6) ou lorsque la durée d'acquisition a dépassé une durée considérée comme pathologique (par exemple 30 secondes), ou encore lorsque le temps entre deux images consécutives à afficher dépasse un temps défini considéré comme significatif d'une homogénéisation de la perfusion (par exemple 15 secondes). Le signal de fluorescence des perforantes est un signal transitoire qui est vite estompé par l'homogénéisation du signal sur l'ensemble d'une zone de tissu biologique telle qu'un lambeau de l'artère perforante épigastrique inférieure profonde.

On notera qu'il n'est plus utile de remplir la mémoire tampon circulaire, car chaque nouvelle image de fluorescence Ii(Yj) est sommée à l'intégrale précédente des signaux de fluorescence, et ceci jusqu'à la fin de l'acquisition des images de fluorescence, prédéterminée pour l'observation envisagée.

Autrement dit, après T₀, à chaque fois qu'une image de fluorescence Ii(Yj) extraite de la vidéo est ajoutée aux précédentes, le logiciel réalise les opérations suivantes :
- L'image de fluorescence Ii(Yj) extraite de la vidéo est recallée par rapport aux précédentes, éventuellement l'image I_{Ref} est soustraite.
- L'image de fluorescence ainsi recallée est sommée aux précédentes, pour obtenir une nouvelle image intégrée I'(Yj) correspondant à la somme des images de fluorescence de T₀⁻ΔT à Tⱼ (avec ΔT=3 secondes dans l'exemple présenté ci-dessus).
- L'image intégrée I'(Yj) est normalisée par rapport au maximum d'intensité obtenu sur cette image I'(Yj). On obtient alors une image I'_{NORM}(Yj).
- L'intensité de chaque pixel de l'image intégrée et normalisée I'_{NORM}(Yj) est seuillée.
- La proportion P(Yj)% de pixels dont l'intensité associée, après intégration et normalisation, est supérieure au pourcentage X% est calculée et comparée à une valeur Yj et si P(Yj)% est supérieure ou égale à Yj, l'image I'_{NORM}(Yj) est colorisée et archivée, sinon les images Ii(Yj) continuent d'être sommées aux précédentes jusqu'à l'obtention d'une image I'_{NORM}(Yj) ayant une proportion P(Yj)% supérieure ou égale à Yj. L'image courante, correspondant au temps Tj auquel la dernière image Ii(Yj) a été extraite de la vidéo, est également archivée.
- Lorsqu'un ensemble d'images I'_{NORM}(Yj) avec j=0 à Z représentatif du phénomène étudié est obtenu, les images I'_{NORM}(Yj) colorisées sont affichées, superposées chacune respectivement sur l'image courante correspondante (Etape 700).

Ainsi, ne sont affichées que des images I'_{NORM}(Yj) présentant une surface de diffusion correspondant un pourcentage P(Yj)% de pixels supérieur ou égal à Yj%, un niveau d'intensité relative supérieure à X% étant fixé pour déterminer si un pixel doit être pris en compte dans le calcul de la surface de diffusion.

Les images de fluorescence sont donc extraites de la vidéo avec une fréquence F. Les données correspondant à ces images de fluorescence captées par la caméra de fluorescence et extraites de la vidéo sont transmises à l'ordinateur 2, puis sont recalées et sommées en continu, mais seules sont colorisées et mises en mémoire, des images intégrées correspondant à des surfaces déterminées de diffusion du produit fluorescent sur la zone de tissu biologique d'intérêt.

Le logiciel archive de cette manière Z images. Dans l'exemple présenté dans ce document Z = 6. Ces six images correspondent respectivement à Y0 = 5% de surface, Y1= 10% de surface, Y2= 20% de surface, Y3= 30% de surface, Y4=40% de surface et Y5= 50% de surface). Ces images sont calculées et archivées avec un tempo qui dépend de la vitesse de diffusion du marqueur chez le patient considéré et non d'intervalles de temps prédéfinis. Ces images sont représentées sur la ligne inférieure de la figure 5.

Selon une variante, le logiciel réalise notamment les opérations suivantes :
- Extraction d'une image de fluorescence Ii(Yk) de la vidéo,
- Recalage de l'image de fluorescence Ii(Yk) (avec éventuellement soustraction d'une image de référence I_{Ref}),
- Somme de l'image de fluorescence Ii(Yk) avec la somme des images de fluorescence déjà calculées (par exemple depuis T₀-ΔT à Tₖ₋₁, soit de T₀-3s à Tₖ₋₁ pour l'exemple présenté ci-dessus) pour obtenir une nouvelle image de fluorescence intégrée I'(Yk),
- Normalisation de l'image intégrée I'_{NORM}(Yk),
- Seuillage des pixels de l'image normalisée supérieurs à un seuil défini,
- Comptage de la proportion P(Yk)% de pixels supérieurs à ce seuil,
- Colorisation de cette image normalisée,
- Mise en mémoire de cette image colorisée, avec la proportion de pixels supérieurs au seuil, associée à cette image,
- Mise en mémoire de l'image courante obtenue de manière concomitante à l'extraction de l'image Ii(Yk) de la vidéo.

Selon cette variante, les images de fluorescence sont donc extraites de la vidéo avec une fréquence F. Les données correspondant à ces images de fluorescence captées par la caméra infra-rouge et extraites de la vidéo sont transmises à l'ordinateur 2, puis sont recalées et sommées en continu, avant d'être normalisées, colorisées et mises en mémoire. Mais, toutes les images Ii(Yk) sont archivées en y associant la proportion P(Yk)% de pixels dont l'intensité associée, après intégration et normalisation, est supérieure au pourcentage X%, c'est-à-dire avec une grandeur représentative de la surface de diffusion correspondante. Le choix des images à afficher se fait postérieurement, en sélectionnant les surfaces de diffusion jugées pertinentes pour l'application envisagée.

Selon une autre variante, les images de fluorescence sont extraites de la vidéo avec une fréquence F. Les données correspondant à ces images de fluorescence captées par la caméra de fluorescence et extraites de la vidéo sont transmises à l'ordinateur 2, mais sont recalées et sommées à la fin du processus d'acquisition, avant d'être normalisées, colorisées et mises en mémoire.

Par ailleurs, en fin d'acquisition, le logiciel analyse l'ensemble des Z images courantes archivées (dans le cas où celles-ci sont réalisées dans la gamme de longueurs d'ondes détectées par la caméra de fluorescence). Le logiciel détermine le maximum de la valeur du signal représentatif de l'intensité de l'émission de fluorescence associée à chaque pixel sur l'ensemble de ces images. Le logiciel normalise chaque image par rapport à ce maximum. Le logiciel colorise chaque image ainsi normalisée en utilisant une autre table de conversion de couleurs que celle pour les images de fluorescence sommées (intégrées).

L'ensemble des images de fluorescence sommées (intégrées) et des images courantes obtenues et mises en mémoire sont affichées ensemble sur un écran (Figure 5), par exemple sous forme de deux bandes horizontales :les images correspondant aux différents pourcentages de la surface de diffusion par rapport à l'ensemble de la surface de la zone de tissu biologique observée (pour l'exemple illustré : Y0 = 5% de surface, Y1= 10% de surface....Y5= 50% de surface) étant placées respectivement l'une au-dessus de l'autre.

La ligne inférieure correspond à un exemple d'images de fluorescence obtenues après le traitement mentionné ci-dessus. Pour plus de clarté, on donne par exemple un effet de transparence en colorisant uniquement les valeurs du signal correspondant à plus de X%=50% de la valeur maximale de ce signal (il est possible d'utiliser d'autres valeurs de X%, X%=30% par exemple peut être envisagé pour certaines applications). Par exemple, les signaux correspondant à 50% de ce maximum correspondent à la couleur jaune, les signaux correspondant à 70% de ce maximum correspondent à la couleur orange et les signaux correspondant à 90% de ce maximum correspondent à la couleur rouge. Par contre les valeurs du signal correspondant à moins de X%=50% de la valeur maximale de ce signal restent en niveaux de gris (alternativement ces valeurs peuvent être affichées en transparence sur l'image courante ou sur l'image de référence I_{Ref} en niveaux de gris). Une représentation de ce type permet de mettre en évidence, pour le praticien, les zones de la zone de tissu biologique observée, dans lesquelles la montée du signal de fluorescence est plus rapide. Ainsi, le praticien peut visuellement mieux repérer et localiser les perforantes potentielles.

Une représentation du type de celle correspondant à la ligne supérieure de la figure 5 permet de mettre en évidence, pour le praticien, l'état de perfusion de l'ensemble de la zone de tissu biologique observée. Ainsi, le praticien peut visuellement constater d'éventuels problèmes de perfusions.

D'une manière plus générale, les images résultant de la normalisation et de la colorisation, peuvent être soit affichées telles quelles, soit en transparence sur l'image courante ou sur l'image de référence I_{Ref}.

Ainsi, les signaux inférieurs à un seuil sont:
- soit laissés en niveaux de gris
- soit non affichés et à la place sont remplacés, soit par les niveaux de gris de l'image courante ; soit par les niveaux de gris de l'image de référence I_{Ref}.
- soit affichés en transparence sur l'image courante ou sur l'image de référence I_{Ref}.

Le logiciel permet également au praticien (si la caméra n'a pas, ou peu, bougé) de superposer, de préférence en transparence, une image de fluorescence normalisée, ou vignette, colorisée (par exemple en ne superposant que les pixels colorisés, c'est à dire correspondant à des valeurs du signal correspondant à plus de X% de la valeur maximale de ce signal, par exemple avec X%=50%) sur l'image « directe » ou de contexte (dite « live en anglais ») correspondant à une image obtenue en éclairant la zone de tissu biologique à observer avec des diodes électroluminescentes émettant dans la gamme de longueurs d'ondes sur laquelle la caméra est sensible (par exemple, le proche infra-rouge), ou bien avec la lumière d'excitation car en fin de perfusion, la fluorescence est généralement homogène. Cela constitue une aide à la localisation des perforantes. Les vignettes obtenues plutôt en début de perfusion (voir aussi les figures 6 et 7) permettent une localisation plus précise, car à la fin de l'acquisition le tissu étant généralement perfusé correctement et de manière homogène, la fluorescence est répartie sur l'ensemble de la zone de tissu observée.

Sur notre exemple, si le praticien souhaite superposer la deuxième vignette en partant de la gauche sur la ligne inférieure de la figure 5, sur l'image « directe », le logiciel lui permet de la superposer en transparence, sur l'image « directe », obtenant ainsi en temps réel l'image correspondant à la figure 6. Il peut alors directement aller pointer la perforante, sur la zone de tissu biologique observée, avec un stylo, pour faire une marque sur le lieu de cette perforante (voir la flèche sur la figure 6).

Si le praticien souhaite superposer la troisième vignette en partant de la gauche sur la ligne inférieure de la figure 5, sur l'image « directe », le logiciel lui permet de la superposer, sur l'image « directe », obtenant ainsi en temps réel l'image correspondant à la figure 7. Il peut alors localiser une deuxième perforante qui peut servir à perfuser une autre moitié d'un lambeau de l'artère perforante épigastrique inférieure profonde par exemple (voir la flèche sur la figure 7).

Afficher plusieurs vignettes permet notamment de ne pas imposer de résultat au praticien et de le laisser choisir une image en fonction du geste qu'il veut réaliser et de l'information qu'il souhaite avoir. L'affichage de l'ensemble des images lui fournit une représentation visuelle d'une évolution dynamique.

Grâce à l'invention, un praticien peut visualiser des images plutôt que des courbes, ce qui facilite l'interprétation. En outre, les images peuvent être colorisés pour faire ressortir les paramètres importants à interpréter. Le procédé selon l'invention, grâce aux calculs mis en œuvre, permet de faire ressortir des paramètres spécifiques des perforantes (signal qui monte vite et haut en intensité). Le procédé selon l'invention permet de se focaliser sur une échelle de temps qui permet de faire ressortir les caractéristiques des perforantes. En effet, en réalisant des observations sur une échelle de temps trop large et en ne se recentrant pas sur le tout début de l'apparition du signal de fluorescence, on perd de l'information et de la précision sur les perforantes.

D'une manière plus générale, grâce au procédé selon l'invention, au moins deux images successives d'une séquence, mais préférentiellement plusieurs images successives dans une séquence, peuvent être comparées, pour être interprétées visuellement par un praticien, sur la base d'informations présentées sous forme d'images ou de vignettes. Les paramètres nécessaires à cette interprétation sont directement mis en évidence sur les images et par la comparaison visuelle des images. La dynamique de diffusion de la fluorescence (donc de la perfusion) est visible par superposition (overlay ou transparence) avec une image « directe » de la zone de tissu biologique étudiée, afin de localiser avec précision, dans ce tissu, les zones où le signal monte en premier et plus rapidement. Les images sont recalées les unes par rapport aux autres automatiquement, ce qui permet d'obtenir des résultats plus fiables. Notamment, on peut observer des scènes qui peuvent légèrement bouger dans le temps (par exemple du fait du mouvement d'un lambeau de l'artère perforante épigastrique inférieure profonde avec la respiration).

Le procédé peut être mis en œuvre sur un ordinateur, à l'aide d'un logiciel qui automatise l'acquisition des résultats, sans que l'intervention d'un chirurgien soit nécessaire. En outre, l'absence d'intervention d'un praticien et l'absence de choix à réaliser pour obtenir les résultats, limite, voire évite, les erreurs d'interprétation et rend la mesure reproductible.

Le procédé et le dispositif selon l'invention permettent de gérer des scènes qui peuvent bouger légèrement dans le temps.

Le procédé et le dispositif selon l'invention, en combinaison avec l'affichage d'une image « directe » aident le chirurgien à se repérer précisément en superposant les résultats calculés, sur cette image. En effet, un repérage serait autrement difficile du fait qu'il y a généralement peu ou pas de repères anatomiques sur les zones de tissus biologique observé, notamment lors de la recherche de perforantes. En outre, le signal de fluorescence devient homogène au bout de quelques secondes, ce qui rend rapidement tout repérage impossible, s'il n'est pas réalisé en temps réel, au début de la perfusion du fluorophore.

Dans l'exemple de mise en œuvre du procédé décrit ci-dessus en relation avec la figure 3 notamment, une seule image supplémentaire est sommée à celles déjà sommées. Selon une variante, plusieurs images de fluorescence acquises peuvent être recalées entre elles et sommées à celles déjà sommées.

On peut aussi décrire un exemple de procédé selon l'invention de la manière suivant. Il comprend :
- l'association à un temps Ti d'une série de n images de fluorescence, avec n= 1 à N, capturées à l'aide de la caméra de fluorescence pendant un intervalle de temps ΔTi, dépendant de la surface couverte par des pixels dont la valeur est supérieure à un seuil donné,
- l'association à un temps Tj, postérieur au temps Ti, d'une série de m images de fluorescence avec m= 1 à M, capturées à l'aide de la caméra infra-rouge pendant un intervalle de temps ΔTj, dépendant également de la surface couverte par des pixels dont la valeur est supérieure à un seuil donné.
- le recalage entre elles des n et m images de fluorescence acquises et associées respectivement au temps Ti et Tj.

Puis d'une part, les n images de fluorescence associées au temps Ti et recalées sont sommées entre elles, pixel par pixel, pour obtenir une image de fluorescence I(Yi), qui est ensuite elle-même sommée à une image obtenue par sommation d'images capturées à des instants antérieurs à Ti, l'image résultante correspondant alors à une image I'(Yi).

D'autre part, les m images de fluorescence associées au temps Tj et recalées entre elles sont sommées entre elles, pixel par pixel, pour obtenir une image de fluorescence I(Yj). On notera, que dans l'exemple de mise en œuvre décrit ci-dessus, m=1 et I(Yj) ne correspond qu'à une seule image de fluorescence.

Les images de fluorescence I'(Yi) et I(Yj) sont ensuite sommées pour obtenir une image de fluorescence sommée I'(Yj).

Cette image est normalisée pour donner I'_{NORM}(Yj). Elle est ensuite seuillée et si le pourcentage P(Yj)% de pixels correspondant à une intensité du signal au-dessus d'un seuil X% a suffisamment varié par rapport à ce qu'il était pour l'image I'_{NORM}(Yi), l'image I'_{NORM}(Yj) est colorisée en vue, par exemple, de la superposer (overlay) à une image directe vue par la caméra. Par exemple, cette image directe est l'image captée en temps réel ou l'image directe captée à Tj. Un effet de transparence peut être obtenu par pondération des canaux RGB, permettant de conserver un certain pourcentage des valeurs de ces canaux dans l'image directe, à laquelle s'ajoute le pourcentage complémentaire à 100% de la colorisation déterminée précédemment. On tire avantage de cet effet de transparence pour voir le stylo ou l'outil du chirurgien à travers l'image en transparence (overlay).

Lorsqu'on se réfère dans les paragraphes précédents à des temps Ti et Tj, c'est essentiellement pour préciser l'ordre temporel des acquisitions d'images, mais le paramètre pertinent auquel on se réfère en tant qu'information utile au praticien, est le nombre de pixels représentant une valeur du signal supérieure ou égale à un certain seuil. Autrement dit, l'information utile est liée à la surface de tissu biologique sur laquelle on peut observer la diffusion du marqueur.

Le procédé selon l'invention permet de prendre automatiquement en considération la variabilité entre patients pour faciliter l'accès aux informations pertinentes et optimiser l'interprétation des informations relatives à la dynamique de diffusion du signal de fluorescence pour les praticiens. L'analyse de ces informations est très simple, puisque principalement basée sur des images et facilement localisables sur le patient.

On notera que l'on peut être intéressé aussi bien par une augmentation du pourcentage de pixels correspondant à une intensité du signal au-dessus du seuil, qu'à une diminution de ce pourcentage. Le suivi d'une augmentation de ce nombre de pixels peut être utile pour caractériser l'apparition et la diffusion du marqueur fluorescent dans la zone de tissu biologique étudiée. Le suivi de la diminution de ce nombre de pixels peut être utile pour d'autres applications (par exemple pour quantifier l'évacuation du traceur fluorescent et évaluer le retour veineux).

On notera que le procédé selon l'invention permet une utilisation de la sonde 1 avec ou sans bras.

Dans ce document, le verbe « sommer » ou les mots « sommation » ou « somme » correspondent à
- des sommes simples (par exemple I(Yi) + I(Yj)),
- des sommes pondérées (par exemple a^{∗}I(Yi) + b^{∗}I(Yj) avec a et b des facteurs de pondérations qui pourraient notamment mettre l'accent sur le fait que l'image I(Yi) arrive en premier par rapport à l'image I(Yj), etc...),
- des sommes pondérées par pixel (par exemple pour donner plus de poids aux pixels correspondant à des variations rapides d'intensité entre images consécutives), dans ce cas on étudie la variation par pixel entre deux images consécutives et on construit une matrice de pondération afin de donner plus de poids aux pixels qui ont subi les plus fortes augmentations d'intensités entre les deux images,
- une somme des différences d'intensité par pixel entre deux images, etc.

## Revendications

1. Procédé de suivi de la diffusion d'un marqueur fluorescent au sein d'un tissu biologique (20), ce procédé comprenant au moins une acquisition d'images de fluorescence, à l'aide d'une caméra, chaque acquisition d'images de fluorescence comprenant la capture et la mise en mémoire d'au moins une image de fluorescence sur une zone du tissu biologique (20) et chaque image de fluorescence correspondant à un ensemble de pixels, une valeur relative d'un signal représentatif de l'intensité de l'émission de fluorescence en un point de la zone du tissu biologique (20) étant associée à au moins certains pixels,
**caractérisé par le fait qu'**il comprend, au cours d'une même perfusion du marqueur fluorescent, une étape consistant à déterminer automatiquement, après comparaison de la surface de diffusion du marqueur biologique mesurée sur au moins deux images de fluorescence, si tout ou partie d'au moins l'une de ces images de fluorescence pour lesquelles la surface de diffusion a été mesurée doit être affichée.

2. Procédé selon la revendication 1, dans lequel la surface de diffusion du marqueur biologique est mesurée en calculant un nombre de pixels correspondant à une intensité du signal supérieure à un seuil prédéfini.

3. Procédé selon la revendication 2, dans lequel on calcule, pour une zone du tissu biologique, la proportion du nombre de pixels correspondant à une intensité du signal supérieure au seuil prédéfini, par rapport au nombre total de pixels de cette zone.

4. Procédé selon l'une des revendications précédentes, dans lequel tout ou partie d'une image de fluorescence pour laquelle la surface de diffusion a été mesurée est affichée,
- si la surface de diffusion mesurée est supérieure ou égale à une valeur prédéfinie, lorsqu'on souhaite caractériser une augmentation de la diffusion du marqueur fluorescent au sein du tissu biologique, ou
- si la surface de diffusion mesurée est inférieure ou égale à une valeur prédéfinie, lorsqu'on souhaite caractériser une diminution de la diffusion du marqueur fluorescent au sein du tissu biologique.

5. Procédé selon l'une des revendications précédentes, dans lequel dans une image de fluorescence pour laquelle il a été déterminé qu'elle doit être affichée, la valeur relative du signal associée à au moins certains pixels correspond à une valeur calculée par intégration, depuis un instant antérieur ou égal à un temps défini comme étant représentatif du début de la diffusion, de la valeur relative du signal sur chacun de ces pixels.

6. Procédé selon la revendication 5, dans lequel, après intégration, la valeur relative du signal associée à au moins certains pixels est normalisée pour tout ou partie des pixels d'une l'image de fluorescence à afficher.

7. Procédé selon la revendication 6, dans lequel, après avoir été normalisée, la valeur relative du signal associée à au moins certains pixels est seuillée.

8. Procédé selon la revendication 7, dans lequel la surface de diffusion du marqueur biologique est mesurée à partir d'une proportion du nombre de pixels
- supérieure ou égale à une valeur prédéfinie, lorsqu'on souhaite caractériser une augmentation de la diffusion du marqueur fluorescent au sein du tissu biologique, ou
- inférieure ou égale à une valeur prédéfinie, lorsqu'on souhaite caractériser une diminution de la diffusion du marqueur fluorescent au sein du tissu biologique.

9. Procédé selon l'une des revendications précédentes, comprenant les étapes suivantes :
- Etape A : une série de n images de fluorescence, avec n= 1 à N, est capturée à l'aide de la caméra et transmise à un ordinateur qui réalise les étapes suivantes, à partir des valeurs du signal représentatif de l'intensité de l'émission de fluorescence et associé à un pixel,
- Etape B : si n est supérieur à 1, la valeur relative du signal associée à chaque pixel d'une image de la série des n images est sommée avec les autres valeurs relatives de signal associées à ce même pixel dans chacune des autres images capturées de cette série, pour obtenir une image I(Yi), sinon, si n=1 et l'image I(Yi) correspond à l'image unique obtenue à l'étage A,
- Etape C : l'image I(Yi) est sommée, de pixel à pixel, à une image correspondant à une somme d'images déjà calculée, pour obtenir une image I'(Yi),
- Etape D : un traitement numérique est réalisé sur la valeur relative du signal associée à chaque pixel d'au moins une zone de l'image I'(Yi) de manière
- - à déterminer la proportion du nombre de pixels correspondant à une intensité du signal supérieure à un seuil prédéfini, par rapport au nombre total de pixels de cette zone,
- - à comparer cette proportion du nombre de pixels à une valeur prédéfinie et
- à archiver l'image I'(Yi) si cette proportion du nombre de pixels a dépassé la valeur prédéfinie, soit lors d'une augmentation, soit lors d'une diminution, de cette proportion.

10. Procédé selon la revendication 9, dans lequel l'étape D comprend la normalisation de l'image I'(Yi) par rapport au maximum d'intensité du signal de fluorescent déterminé pour cette image, afin d'obtenir une image normalisée I'_{NORM}(Yi) dont les valeurs de signal associées respectivement à chaque pixel sont comprises entre 0% et 100%, c'est alors l'image I'_{NORM}(Yi) qui est archivée.

11. Procédé selon la revendication 10, dans lequel au moins deux images de fluorescence normalisée I'_{NORM} (Yi) et I'_{NORM} (Yj) sont traitées et affichées de manière à être vues simultanément.

12. Procédé selon la revendication 10 ou 11, dans lequel l'image I'_{NORM}(Yi) est affichée superposée, en transparence, à une image directe de la zone de tissu biologique vue par une caméra adaptée pour capturer l'image directe.

13. Procédé selon la revendication 12, dans lequel on superpose uniquement les valeurs du signal de l'image I'_{NORM}(Yi) supérieures à un seuil prédéterminé correspondant à un pourcentage prédéfini de la valeur maximale de ce signal.

14. Procédé selon l'une des revendications précédentes, dans lequel une intensité moyenne (M_{(i,j)} ou M₍ₖ₎) est calculée pour des images de fluorescence, ou pour au moins une zone de ces images de fluorescence, puis est comparée à une intensité moyenne (M_{init(i,j} ou Mᵢₙᵢₜ₍ₖ₎) calculée pour au moins une image de fluorescence d'origine, ou pour la zone correspondant à l'intensité moyenne, dans chaque image de fluorescence d'origine, pour déterminer un seuil au-delà duquel il peut être considéré que l'émission de fluorescence dans la zone du tissu biologique (20) est représentative d'un effet d'une perfusion d'un marqueur injecté dans le tissu biologique (20) à observer.

15. Dispositif de suivi de la fluorescence émise à la surface du tissu biologique, comprenant :
- une sonde (1) avec une caméra pour capturer des images de fluorescence, cette sonde (1) comprenant elle-même une source d'excitation adaptée pour émettre un rayonnement d'excitation d'un marqueur de fluorescence et un capteur de fluorescence pour détecter une lumière de fluorescence émise à la surface du tissu biologique (20),
- un ordinateur (2) programmé pour enregistrer et stocker des images de fluorescence capturées par la caméra et réaliser un traitement des images de fluorescence, à l'aide d'un logiciel pour la mise en œuvre du procédé selon l'une des revendications précédentes, cet ordinateur comprenant des moyens de mémoire pour enregistrer et stocker des valeurs correspondant à une intensité d'un signal de fluorescence émis par la zone de tissu biologique, et associées aux pixels respectifs de chaque image de fluorescence, et des moyens de calcul pour recaler, normaliser et sommer pixel par pixel au moins deux images de fluorescence et coloriser l'image de fluorescence sommée résultante, et
- des moyens d'affichage (3) pour afficher au moins une image de fluorescence sommée et colorisée.

16. Dispositif selon la revendication 15, comprenant un capteur optique pour détecter une lumière dans le spectre visible et capturer une image directe sur laquelle est superposée une image de fluorescence résultant d'au moins une étape de sommation avec une ou plusieurs autres images.

## Patentansprüche

1. Verfahren zur Überwachung der Diffusion eines fluoreszierenden Markers innerhalb eines biologischen Gewebes (20), wobei das Verfahren mindestens eine Erfassung von Fluoreszenzbildern mithilfe einer Kamera umfasst, wobei jede Erfassung von Fluoreszenzbildern die Erfassung und die Speicherung mindestens eines Fluoreszenzbildes in einem Bereich von biologischem Gewebe (20) umfasst und jedes Fluoreszenzbild einem Satz von Pixeln entspricht, wobei ein relativer Wert eines Signals, das für die Stärke der Fluoreszenzemission an einem Punkt des Bereichs des biologischen Gewebes (20) repräsentiv ist, mindestens bestimmten Pixeln zugeordnet ist,
**dadurch gekennzeichnet, dass** es im Verlauf einer selben Perfusion des fluoreszenten Markers einen Schritt umfasst, der darin besteht, nach Vergleich der Diffusionsoberfläche des biologischen Markers, die auf mindestens zwei Fluoreszenzbildern gemessen wird, automatisch zu bestimmen, ob das gesamte oder ein Teil von mindestens einem der Fluoreszenzbilder, für die die Diffusionsoberfläche gemessen wurde, angezeigt werden muss.

2. Verfahren nach Anspruch 1, wobei die Diffusionsoberfläche des biologischen Markers gemessen wird, indem eine Anzahl von Pixeln berechnet wird, die einer Stärke des Signals über einem vordefinierten Schwellenwert entspricht.

3. Verfahren nach Anspruch 2, wobei für einen Bereich des biologischen Gewebes der Anteil der Anzahl von Pixeln, der einer Stärke des Signals über dem vordefinierten Schwellenwert entspricht, im Verhältnis zur Gesamtanzahl von Pixeln dieses Bereichs berechnet wird.

4. Verfahren nach einem der vorhergehenden Ansprüche, wobei das gesamte oder ein Teil von einem Fluoreszenzbild, für das die Diffusionsoberfläche gemessen wurde, angezeigt wird,
- wenn die gemessene Diffusionsoberfläche größer oder gleich einem vordefinierten Wert ist, wenn eine Zunahme der Diffusion des fluoreszenten Markers innerhalb des biologischen Gewebes charakterisiert werden soll, oder
- wenn die gemessene Diffusionsoberfläche kleiner oder gleich einem vordefinierten Wert ist, wenn eine Abnahme der Diffusion des fluoreszenten Markers innerhalb des biologischen Gewebes charakterisiert werden soll.

5. Verfahren nach einem der vorhergehenden Ansprüche, wobei in einem Fluoreszenzbild, für bestimmt wurde, dass es angezeigt werden muss, der relative Wert des Signals, der mindestens bestimmten Pixeln zugeordnet ist, einem Wert entspricht, der durch Integralrechnung ab einem Zeitpunkt vor oder gleich einer definierten Zeit, die als repräsentativ für den Beginn der Diffusion definiert ist, für den relativen Wert des Signals auf jedem der Pixel berechnet wird.

6. Verfahren nach Anspruch 5, wobei nach der Integralrechnung der relative Wert des Signals, der mindestens bestimmten Pixeln zugeordnet ist, für die gesamten oder einen Teil der Pixel eines anzuzeigenden Fluoreszenzbildes normiert wird.

7. Verfahren nach Anspruch 6, wobei nach der Normierung der relative Wert des Signals, der mindestens bestimmten Pixeln zugeordnet ist, mit Schwellenwerten unterlegt versehen ist.

8. Verfahren nach Anspruch 7, wobei die Diffusionsoberfläche des biologischen Markers auf der Grundlage eines Anteils der Anzahl von Pixeln gemessen wird als
- größer oder gleich einem vordefinierten Wert, wenn eine Zunahme der Diffusion des fluoreszenten Markers innerhalb des biologischen Gewebes charakterisiert werden soll, oder
- kleiner oder gleich einem vordefinierten Wert, wenn eine Abnahme der Diffusion des fluoreszenten Markers innerhalb des biologischen Gewebes charakterisiert werden soll.

9. Verfahren nach einem der vorhergehenden Ansprüche, umfassend die folgenden Schritte:
- Schritt A: eine Serie von n Fluoreszenzbildern mit n=1 bis N wird mithilfe der Kamera erfasst und an einen Computer übertragen, der auf der Grundlage der Werte des Signals, das für die Stärke der Fluoreszenzemission repräsentativ ist und einem Pixel zugeordnet ist, die folgenden Schritte realisiert,
- Schritt B: wenn n größer als 1 ist, wird der relative Wert des Signals, der jeden Pixel eines Bildes der Serie der n Bildern zugeordnet ist, mit den anderen relativen Signalwerten summiert, die demselben Pixel in jedem der anderen erfassten Bilder dieser Serie zugeordnet sind, um ein Bild I(Yi) zu erhalten, andernfalls, wenn n=1 und das Bild I(Yi) dem einzigen, in Schritt A erhaltenen Bild entspricht,
- Schritt C: das Bild I(Yi) wird Pixel für Pixel mit einem Bild summiert, das einer Summe bereits berechneter Bilder entspricht, um ein Bild I'(Yi) zu erhalten,
- Schritt D: eine digitale Verarbeitung wird an dem relativen Wert des Signals, der jedem Pixel von mindestens einem Bereich des Bildes I'(Yi) zugeordnet ist, durchgeführt, um
- den Anteil der Anzahl von Pixeln, die einer Stärke des Signals über einem vordefinierten Schwellenwert entsprechen, im Verhältnis zur Gesamtzahl von Pixeln dieses Bereichs zu bestimmen,
- diesen Anteil der Anzahl von Pixeln mit einem vordefinierten Wert zu vergleichen und
- das Bild I'(Yi) zu archivieren, wenn dieser Anteil der Anzahl von Pixeln den vordefinierten Wert dieses Anteils überschritten hat, sei es bei einer Zunahme, sei es bei einer Abnahme.

10. Verfahren nach Anspruch 9, wobei Schritt D die Normierung des Bildes I'(Yi) im Verhältnis zum Maximum der Stärke des Fluoreszenzsignals umfasst, das für dieses Bild bestimmt wurde, um ein normiertes Bild I'_{NORM}(Yi) zu erhalten, dessen Signalwerte, die jedem Pixel jeweils zugeordnet sind, zwischen 0 % und 100 % liegen, und dann das Bild I'_{NORM}(Yi) archiviert wird.

11. Verfahren nach Anspruch 10, wobei mindestens zwei normierte Fluoreszenzbilder I'_{NORM}(Yi) und I'_{NORM}(Yj) derart verarbeitet und angezeigt werden, dass sie gleichzeitig zu sehen sind.

12. Verfahren nach Anspruch 10 oder 11, wobei das Bild I'_{NORM}(Yi) transparent über ein direktes Bild des Bereichs von biologischem Gewebe, das von einer zur Erfassung des direkten Bildes eingerichteten Kamera gesehen wird, gelegt wird.

13. Verfahren nach Anspruch 12, wobei nur die Signalwerte des Bildes I'_{NORM}(Yi) über einem vorbestimmten Schwellenwert, der einem vordefinierten Prozentsatz des Maximalwertes dieses Signals entspricht, darübergelegt werden.

14. Verfahren nach einem der vorhergehenden Ansprüche, wobei eine mittlere Stärke (M_{(i,j)} oder M₍ₖ₎) für Fluoreszenzbilder oder für mindestens einen Bereich dieser Fluoreszenzbilder berechnet wird, dann mit einer mittleren Stärke (M_{init(i,j)} oder Mᵢₙᵢₜ₍ₖ₎) verglichen wird, die für mindestens ein ursprüngliches Fluoreszenzbild oder für den Bereich, der der mittleren Stärke entspricht, in jedem ursprünglichen Fluoreszenzbild berechnet wird, um einen Schwellenwert zu bestimmen, jenseits dessen davon ausgegangen werden kann, dass die Fluoreszenzemission im Bereich des biologischen Gewebes (20) für eine Wirkung einer Perfusion eines in das zu beobachtende biologische Gewebe (20) eingespritzten Markers repräsentativ ist.

15. Vorrichtung zur Überwachung der Fluoreszenz, die an der Oberfläche des biologischen Gewebes emittiert wird, umfassend:
- eine Sonde (1) mit einer Kamera zur Erfassung von Fluoreszenzbildern, wobei diese Sonde (1) selbst eine Erregungsquelle, die dafür eingerichtet ist, eine Erregungsstrahlung eines Fluoreszenzmarkers zu emittieren, und einen Fluoreszenzsensor umfasst, um ein Fluoreszenzlicht zu erkennen, das an der Oberfläche des biologischen Gewebes (20) emittiert wird,
- einen Computer (2), der zur Aufzeichnung und Speicherung von durch die Kamera erfassten Fluoreszenzbildern und Durchführung einer Verarbeitung von Fluoreszenzbildern zur Umsetzung des Verfahrens nach einem der vorhergehenden Ansprüche mithilfe einer Software programmiert ist, wobei dieser Computer Speichermittel, um Werte aufzuzeichnen und zu speichern, die einer Stärke eines Fluoreszenzsignals entsprechen, das von dem biologischen Gewebebereich emittiert wird, und die den jeweiligen Pixeln jedes Fluoreszenzbildes zugeordnet sind, und Rechenmittel zum Einstellen, Normieren und Summieren mindestens zweier Fluoreszenzbilder Pixel für Pixel und Kolorieren des resultierenden summierten Fluoreszenzbildes umfasst und
- Anzeigemittel (3) zum Anzeigen mindestens eines summierten und kolorierten Fluoreszenzbildes.

16. Vorrichtung nach Anspruch 15, umfassend einen optischen Sensor zum Detektieren eines Lichts im sichtbaren Spektrum und Erfassen eines direkten Bildes, über das ein Fluoreszenzbild gelegt wird, das sich aus mindestens einem Schritt der Summierung mit einem oder mehreren anderen Bildern ergibt.

## Claims

1. Method for monitoring the diffusion of a fluorescent marker within a biological tissue (20), this method comprising at least one operation of acquiring fluorescence images, using a camera, each operation of acquiring fluorescence images comprising capturing and storing at least one image of fluorescence in a region of the biological tissue (20) and each fluorescence image corresponding to a set of pixels, a relative value of a signal that is representative of the intensity of the fluorescence emission at a point in the region of the biological tissue (20) being associated with at least certain pixels, **characterized in that** it comprises, during the same operation of perfusion of the fluorescent marker, a step consisting in automatically determining, after comparing the area of diffusion of the biological marker measured in at least two fluorescence images, whether all or part at least one of these fluorescence images for which the diffusion area has been measured must be displayed.

2. Method according to Claim 1, wherein the area of diffusion of the biological marker is measured by calculating a number of pixels corresponding to a signal intensity higher than a predefined threshold.

3. Method according to Claim 2, wherein the proportion of the number of pixels corresponding to a signal intensity higher than the predefined threshold is calculated for a region of the biological tissue, relative to the total number of pixels in this region.

4. Method according to one of the preceding claims, wherein all or part of a fluorescence image for which the diffusion area has been measured is displayed
- if the measured diffusion area is larger than or equal to a predefined value, when it is desired to characterize an increase in the diffusion of the fluorescent marker within the biological tissue, or
- if the measured diffusion area is smaller than or equal to a predefined value, when it is desired to characterize a decrease in the diffusion of the fluorescent marker within the biological tissue.

5. Method according to one of the preceding claims, wherein in a fluorescence image for which it has been determined that it must be displayed, the relative signal value associated with at least certain pixels corresponds to a value calculated by integration, from a time prior to or equal to a time defined as being representative of the start of diffusion, of the relative signal value in each of these pixels.

6. Method according to Claim 5, wherein, after integration, the relative signal value associated with at least certain pixels is normalized for all or some of the pixels of a fluorescence image to be displayed.

7. Method according to Claim 6, wherein, after having been normalized, the relative signal value associated with at least certain pixels is thresholded.

8. Method according to Claim 7, wherein the area of diffusion of the biological marker is measured on the basis of a proportion of the number of pixels
- higher than or equal to a predefined value, when it is desired to characterize an increase in the diffusion of the fluorescent marker within the biological tissue, or
- lower than or equal to a predefined value, when it is desired to characterize a decrease in the diffusion of the fluorescent marker within the biological tissue.

9. Method according to one of the preceding claims, comprising the following steps:
- Step A: a series of n fluorescence images, where n = 1 to N, is captured using the camera and transmitted to a computer which performs the following steps, on the basis of the values of the signal that is representative of the intensity of the fluorescence emission and is associated with a pixel,
- Step B: if n is greater than 1, the relative signal value associated with each pixel of an image in the series of n images is summed with the other relative signal values associated with this same pixel in each of the other captured images in this series to obtain an image I(Yi); otherwise, if n = 1 and the image I(Yi) corresponds to the only image obtained in stage A,
- Step C: the image I(Yi) is summed, pixel by pixel, with an image corresponding to a sum of images that has already been calculated, to obtain an image I'(Yi),
- Step D: digital processing is carried out on the relative signal value associated with each pixel of at least one region of the image I'(Yi) so as
- - to determine the proportion of the number of pixels corresponding to a signal intensity higher than a predefined threshold, relative to the total number of pixels in this region,
- - to compare this proportion of the number of pixels with a predefined value and
- to archive the image I'(Yi) if this proportion of the number of pixels has passed the predefined value, either during an increase or during a decrease, of this proportion.

10. Method according to Claim 9, wherein step D comprises normalizing the image I'(Yi) relative to the maximum intensity of the fluorescent signal determined for this image in order to obtain a normalized image I'_{NORM}(Yi), the signal values of which that are associated with each pixel, respectively, are between 0% and 100%; it is then the image I'_{NORM}(Yi) which is archived.

11. Method according to Claim 10, wherein at least two normalized fluorescence images I'_{NORM}(Yi) and I'_{NORM}(Yj) are processed and displayed so as to be viewed simultaneously.

12. Method according to Claim 10 or 11, wherein the image I'_{NORM}(Yi) is displayed superimposed, in a see-through manner, over a direct image of the region of biological tissue viewed by a camera suitable for capturing the direct image.

13. Method according to Claim 12, wherein only the values of the signal of the image I'_{NORM}(Yi) that are higher than a predetermined threshold corresponding to a predefined percentage of the maximum value of this signal are superimposed.

14. Method according to one of the preceding claims, wherein an average intensity (M_{(i,j)} or M₍ₖ₎) is calculated for fluorescence images, or for at least one region of these fluorescence images, and is then compared with an average intensity (M_{init(i,j} or Mᵢₙᵢₜ₍ₖ₎) calculated for at least one original fluorescence image, or for the region corresponding to the average intensity, in each original fluorescence image, to determine a threshold beyond which it can be considered that the emission of fluorescence in the region of the biological tissue (20) is representative of an effect from perfusion of a marker injected into the biological tissue (20) to be observed.

15. Device for monitoring the fluorescence emitted from the surface of the biological tissue, comprising:
- a probe (1) with a camera for capturing fluorescence images, this probe (1) itself comprising an excitation source suitable for emitting excitation radiation for exciting a fluorescence marker and a fluorescence sensor for detecting fluorescence light emitted at the surface of the biological tissue (20),
- a computer (2) programmed to record and to store fluorescence images captured by the camera and to carry out processing of the fluorescence images, using software for implementing the method according to one of the preceding claims, this computer comprising memory means for recording and storing values that correspond to an intensity of a fluorescence signal emitted by the region of biological tissue, and that are associated with the respective pixels of each fluorescence image, and calculation means for aligning, normalizing and summing, pixel by pixel, at least two fluorescence images and colorizing the resulting summed fluorescence image, and
- display means (3) for displaying at least one summed and colorized fluorescence image.

16. Device according to Claim 15, comprising an optical sensor for detecting light in the visible spectrum and capturing a direct image over which a fluorescence image resulting from at least one step of summing with one or more other images is superimposed.
